# EUROPÄISCHE PATENTSCHRIFT

(11) **EP 1 479 389 B1**
(45) Veröffentlichungstag und Bekanntmachung des Hinweises auf die Patenterteilung: **26.09.2007**
(21) Anmeldenummer: 04011907.5
(22) Anmeldetag: 19.05.2004
(51) Int. Cl.: A61K 31/70, A61K 47/08

(54) **Gebrauchsfertige Gemcitabin-Lösungen**
Ready to use gemcitabine solutions
Solutions de gemcitabine prêtes à l'emploi

(30) Priorität: 21.05.2003 DE 10323279
(43) Veröffentlichungstag der Anmeldung: 24.11.2004
(73) Patentinhaber: STADA ARZNEIMITTEL AG, 61118 Bad Vilbel (DE)
(72) Erfinder: Schridde, Edgar, 30451 Hannover (DE); Merbach, Bernd, Dr., 30938 Burgwedel (DE); Gimmel, Stefan-Peter, Dr., 30655 Hannover (DE)
(74) Vertreter: Hamm, Volker

(56) Entgegenhaltungen:
- WO-A2-01/34134
- WO-A2-99/52514
- US-A- 5 464 826

## Beschreibung

Die vorliegende Erfindung betrifft Gemcitabin umfassende pharmazeutische Zusammensetzungen in der Form von gebrauchsfertigen Lösungen.

Gemcitabin (2'-Deoxy-2',2'-difluorcytidin; 1-(4-Amino-2-oxo-1H-pyrimidin-1-yl)-2-deoxy-2,2-difluorribose; dFdC; CAS Nr. 95058-81-4; C₉H₁₁F₂N₃O₄, Mᵣ 263,2) ist eine im Arzneibuch offiziell monographierte Substanz (Official Monographs, USP 27, 1 Supplement USP-NF, page 3060-61 bzgl. "Gemcitabine Hydrochloride" and "Gemcitabine for Injection") und hat die chemische Struktur

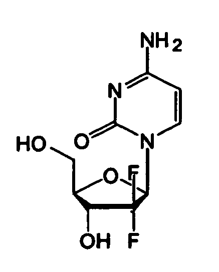

Gemcitabin wurde erstmals in US 4,526,988 beschrieben und kann zur Behandlung von viralen Infektionen oder in der immunosuppressiven Therapie von Autoimmunerkrankungen eingesetzt werden. Die anti-neoplastische Wirksamkeit des Gemcitabins ist in US 5,464,826 offenbart. Es wird beispielsweise allein, aber auch in Kombination mit anderen Zytostatika wie etwa Cisplatin in der Behandlung des lokal fortgeschrittenen oder metastasierten, nichtkleinzelligen Bronchialkarzinoms sowie des fortgeschrittenen Adeno- oder Cysadenokarzinoms des exokrinen Pankreas therapeutisch eingesetzt. Die empfohlene Dosis in der Gemcitabin-Therapie beträgt 1 g / qm Körperoberfläche. Wie andere Nukleosidanaloga kann auch Gemcitabin zytostatisch bei der therapeutischen Behandlung verschiedenster Krebsarten wie z.B. der lymphatischen oder myeloischen Leukämie eingesetzt werden. Dabei erfolgt die Verabreichung von Gemcitabin in der Therapie der verschiedensten Krebserkrankungen intravenös, wodurch es erforderlich wird, den Wirkstoff in Form einer Lösung zur Verfügung zu stellen.

WO-A-99/52514 offenbart intravenöse Formulierungen von Gemcitabin mit einer Wirkstoffkonzentration zwischen 0,0001 mg/ml und 0,025mg/ml.

CN-A-135 29 44 beschreibt lagerstabile, gebrauchsfertige wässrige Lösungen mit einem pH Wert von 4,5 - 9 enthaltend Gemcitabinbase als Wirkstoff, wobei die Gemcitabinkonzentration wenigstens 0,05mg/ml beträgt.

Die zur parenteralen Verabreichung benötigten Gemcitabin-Zubereitungen sind zur Zeit nur in Form von Lyophilisaten (Gemzar^{®}) verfügbar, die vor der Verabreichung an den Patienten rekonstituiert werden müssen. Die Verwendung von solchen gefriergetrockneten Zubereitungen birgt aber wesentliche Nachteile. Zum einen ist das Herstellungsverfahren dieser Lyophilisate kompliziert und teuer, zum anderen stellt die Rekonstitution zusätzliche Arbeitsschritte dar und bedeutet für das damit befasste Personal unerwünschte Risiken. Insbesondere kann es bei der Rekonstituierung der Arzneimittellösungen aus einer Trockensubstanz zum sogenannten "Spray-Back-Effect" kommen, durch den es zu einer weiteren Kontamination und Gefährdung des Personals kommen kann. Demgemäß muss sowohl bei der Herstellung des Lyophilisates, als auch bei seiner Rekonstitution jede Kontamination von Personal oder Inventar mit dem hochwirksamen Zytostatikum vermieden werden. Zudem kann es auch durch andere Fehler in der Handhabung dieser Lyophilisate zu schwerwiegenden Problemen bei der Behandlung mit Gemcitabin kommen, wie z.B. zu einer Abweichung der Wirkstoffkonzentration oder einer mikrobiellen Kontamination der aus dem Lyophilisat hergestellten Lösung.

Aufgrund der vielfältigen Gefahren- und Fehlerquellen bei der Benutzung des lyophilisierten Wirkstoffes zur Herstellung von Gemcitabin-Lösungen war es daher wünschenswert, über gebrauchsfertige, im folgenden auch "ready-to-use-Lösungen" genannte, Arzneimittellösungen zu verfügen.

Bekannterweise sind die gegenwärtig verwendeten, aus Lyophilisaten rekonstituierten Gemcitabin-Lösungen nicht lagerstabil, da sie während ihrer Lagerung einem Abbau des Wirkstoffs unterliegen. Dies führt einerseits zu einer Abweichung der Wirkstoffkonzentration und andererseits zu unerwünschten Verunreinigungen (Abbauprodukten des Gemcitabins) in der Lösung. Derartig verunreinigte Gemcitabin-Lösungen sind aufgrund ihrer möglichen Risiken für den Patienten für Behandlungszwecke nachteilig.

Aufgabe der vorliegenden Erfindung ist es damit, stabile, gebrauchsfertige Gemcitabin-Lösungen zur Verfügung zu stellen, die nicht die zuvor diskutierten Risiken und Nachteile der bekannten Darreichungsformen und daraus hergestellter Lösungen aufweisen. In diesem Zusammenhang bedeutet der Begriff "gebrauchsfertig", dass die Lösung nicht unmittelbar vor der Verabreichung an den Patienten aus einem Feststoff, wie etwa einem kristallinen oder amorphen Feststoff oder einem Lyophilisat, rekonstituiert wurde.

In den der Erfindung zugrundeliegenden Experimenten wurde überraschend gefunden, dass Gemcitabin umfassende gebrauchsfertige Lösungen mit einem pH-Wert in einem Bereich von 3,5 bis 10,0 unerwartet hohe Lagerstabilitäten bei verschiedenen Lagertemperaturen zeigen.

Neben ihren unerwartet hohen Lagerstabilitäten besitzen die erfindungsgemäßen Lösungen den Vorteil, dass sie die bei sauren Gemcitabin-Lösungen, insbesondere die bei solchen mit einem pH-Wert unter 4 auftretenden unerwünschten Nebenwirkungen bei der intravenösen Verabreichung, wie etwa Infusionsschmerz und lokale Unvertäglichkeit, nicht hervorrufen. Die intravenös und lokal besser verträglichen erfindungsgemäßen Gemcitabinlösungen bedeuten somit für den Patienten und den behandelnden Arzt einen großen Vorteil im Sinne einer Verbesserung der Therapieakzeptanz und Therapietreue (Compliance), insbesondere in einer zytostatischen Therapie mit ihren bekannten, wirkstoffbedingten, unerwünschten Nebenwirkungen.

Gemäß einer bevorzugten Ausführungsform liegt der pH-Wert der erfindungsgemäßen Lösungen in einem Bereich von 3,5 bis 10,0. Gemäß einer Ausführungsform zeigen die erfindungsgemäßen Gemcitabin-Lösungen einen pH-Wert von 4,0 bis 5,0; gemäß einer weiteren erfindungsgemäßen Ausführungsform zwischen 7,0 und 10,0; besonders bevorzugt 7,0 bis 9,0. Gemäß einer weiteren, bevorzugten erfindungsgemäßen Ausführungsform stimmt der pH-Wert der Lösung mit dem physiologischen Gewebe- und Blut-pH-Wert von 7,35 bis 7,55 überein. Bei einer besonders bevorzugten erfindungsgemäßen Ausführungsform liegt der pH-Wert der Lösung in einem Bereich von 7,8 bis 8,2.

Die erfindungsgemäßen Lösungen umfassen zwischen 0,05 mg und 16,0 mg Gemcitabin pro ml Lösungsmittel. Vorzugsweise beträgt die Konzentration des Gemcitabins 10 mg/ml. Dabei umfassen die Lösungen ein physiologisch annehmbares Säureadditionssalz, der Gemcitabinbase. Zur Herstellung der erfindungsgemäßen Lösungen wird ein Säureadditionssalz der Gemcitabin-Base mit einer anorganischen Säure, insbesondere Gemcitabinhydrochlorid, verwendet.

Als geeignete Lösungsmittel für erfindungsgemäße Lösungen sind beispielsweise Wasser, Ethanol, Glycerin, 1,2-Propandiol (Propylenglykol), Polyethylenglykol 200-600, Benzylalkohol, Trimethylenglykol, 1,3-Butylenglykol, 2,3-Butylenglykol, Ethylacetat, Ethyllactat, Glykofurol (Tetraglykol), Solketal und Harnstoff zu nennen. Vorzugsweise wird Wasser, Ethanol, Polyethylenglykol 200-600 oder 1,2-Propandiol (Propylenglykol) verwendet, besonders bevorzugt ist Wasser.

Erfindungsgemäß ist es aber auch möglich, den pH-Wert mit mindestens einem physiologisch annehmbaren Ansäuerungs- und/oder Alkalisierungsmittel einzustellen. Beispielsweise sind hierfür geeignet anorganische Säuren und Basen, wie z. B. Salzsäure, Schwefelsäure, schweflige Säure, Salpetersäure, salpetrige Säure, Phosphorsäure, phosphorige Säure, Kohlensäure, Natriumhydroxid, Kaliumhydroxid, Calciumhydroxid und Magnesiumhydroxid; Alkali- und Erdalkalisalze sowie Alkalihydrogen- und Erdalkalihydrogensalze der anorganischen Oxosäuren des Phosphors, Schwefels, Kohlenstoffs und Stickstoffs wie z. B. Natriumphosphat und dessen Hydrate, Natriumhydrogenphosphat und dessen Hydrate, Dinatriumhydrogenphosphat und dessen Hydrate, Dinatriumsulfat, Natriumhydrogensulfat, Natriumsulfit, Calciumsulfit, Magnesiumsulfit, Calciumhydrogencarbonat, Natriumcarbonat, Natriumhydrogencarbonat, Natriumnitrat, Natriumnitrit, Calciumnitrit, Magnesiumnitrat und Magnesiumnitrit; Salze des Chlors wie z. B. Natriumchlorid, Calciumchlorid und Magnesiumchlorid; organische Basen und Säuren wie z. B. Ameisensäure, Essigsäure, Propionsäure, Milchsäure, Oxalsäure, Malonsäure, Maleinsäure, Weinsäure, Zitronensäure, Brenztraubensäure, Benzoesäure, Methansulfonsäure, Ethansulfonsäure, *para*-Toluol-Sulfonsäure, Salicylsäure, Ascorbinsäure, Tris(hydroxymethyl-)aminomethan (2-Amino-2-(hydroxymethyl)-1,3-propandiol; Trometamol; TRIS), 1-Desoxy (methylamino)-D-glucitol (N-Methylglucamin; Meglumin); Alkali- und Erdalkalisalze von organischen Basen und Säuren wie etwa Natriumacetat; sowie Mischungen davon.

Vorzugsweise wird der pH-Wert der erfindungsgemäßen Lösungen eingestellt mit Salzsäure, Phosphorsäure, Schwefelsäure, Natriumhydroxid, Natriumphosphat und dessen Hydraten, Natriumhydrogenphosphat und dessen Hydraten, Dinatriumhydrogenphosphat und dessen Hydraten, Essigsäure, Milchsäure, Zitronensäure, Methansulfonsäure, Ethansulfonsäure, Tris(hydroxymethyl-)aminomethan (Trometamol; TRIS) oder 1-Desoxy(methylamino)-D-glucitol (N-Methylglucamin; Meglumin), insbesondere bevorzugt mit Natriumhydroxid, Salzsäure, Tris(hydroxymethyl-)aminomethan (Trometamol; TRIS) oder 1-Desoxy-(methylamino)-D-glucitol (N-Methylglucamin; Meglumin).

Die Einstellung und/oder Stabilisierung des pH-Wertes kann auch durch einen aus einem aus physiologisch annehmbaren Ansäuerungs- und/oder Alkalisierungsmittel gebildeten Puffer erfolgen. Besonders bevorzugt sind Puffersysteme, wobei der pK-Wert von mindestens einer funktionellen Gruppe der den Puffer bildenden Pufferbase oder Puffersäure innerhalb des Bereiches von pK 2,49 bis 11,01 liegt. Besonders bevorzugte Puffer zur Einstellung des pH-Wertes der erfindungsgemäßen Lösungen sind Natriumacetat, Tris(hydroxymethyl-)aminomethan (Trometamol; TRIS), 1-Desoxy-(methylamino)-D-glucitol (N-Methylglucamin; Meglumin) und Dinatriumhydrogenphosphat oder Mischungen davon.

Die Pufferkonzentration in den erfindungsgemäßen Lösungen liegt zwischen 0,001 g bis 100 g an Pufferkomponente, bevorzugt zwischen 0,05 g bis 20 g an Pufferkomponente und besonders bevorzugt zwischen 0,1 bis 10 g an Pufferkomponente jeweils bezogen auf 1 g Gemcitabin.

Die erfindungsgemäßen Lösungen umfassen gegebenenfalls zusätzlich mindestens einen tonisierenden Hilfsstoff, mindestens einen konservierenden Hilfsstoff und/oder mindestens ein Antioxidationsmittel.

Als Tonizitätsmittel für erfindungsgemäße Lösungen können beispielsweise verwendet werden physiologisch annehmbare anorganische Alkali- oder Erdalkalisalze wie z. B. Natriumchlorid, Calciumchlorid, Magnesiumchlorid, Natriumsulfat, Natriumcarbonat und Calciumhydrogencarbonat; physiologisch annehmbare organische Salze wie z. B. Natriumlactat; physiologisch annehmbare Kohlenhydrate wie z. B. physiologisch annehmbare Cyclodextrine (α-, β-, γ-Cyclodextrine) sowie deren Alkyl- und/oder Aryl-substituierte Derivate, Glucose, Fructose, Sorbitol, Mannitol, Galactose, Inositol, Maltitol, Lactose, Trehalose, Maltose, Saccharose, Dextran 1, Dextran 10, Dextran 40, Dextran 70, Stärke und Hydroxyethylstärke; physiologisch annehmbare Aminosäuren, Peptide oder Proteine wie etwa Glycin, Albumin und Gelatine; sowie Mischungen davon.

Bevorzugte Tonizitätsmittel sind Natriumchlorid, Calciumchlorid, Glucose, Mannitol und Lactose, insbesondere bevorzugt sind Natriumchlorid, Glucose und Mannitol.

Als Konservierungsmittel für erfindungsgemäße Lösungen können beispielsweise verwendet werden Chlorcresol, Benzylalkohol, Ester der *p*-Hydroxybenzoesäure wie etwa Ethylparaben und Methylparaben.

Bevorzugte Konservierungsmittel sind Benzylalkohol, Propylparaben und Methylparaben, insbesondere bevorzugt ist Benzylalkohol.

Als Antioxidationsmittel für die erfindungsgemäßen Lösungen können beispielsweise sauerstoffreduzierende, wie etwa Natriummetabisulfit und Natriumsulfit, oder Übergangsmetallionenbindende, komplex- (chelat-) bildende Antioxidantien, wie z.B. Natriumedetat, verwendet werden.

Erfindungsgemäß ist es möglich, gegebenenfalls mindestens eine weitere Arzneimittellösung zu der ready-to-use-Lösung hinzuzumischen. Beispielsweise kann eine erfindungsgemäße Gemcitabin-Lösung mit einer Cisplatin-Lösung vermischt werden, um einen sogenannten Zytostatikainfusionscocktail zu erhalten.

Die nachfolgenden Beispiele verdeutlichen die Erfindung.

### Beispiel 1

Zur Bestimmung der Löslichkeit von Gemcitabin in wässrigen Lösungen (bei T = 25°C und T = 2-8°C, Anfangs-pH-Wert = 4,0) wurde Gemcitabinhydrochlorid in Wasser für Injektionszwecke bei 25°C gelöst (c(Gemcitabin) = 55,2 mg/ml entsprechend 62,8 mg Gemcitabinhydrochlorid pro ml Wasser sowie c(Gemcitabin) = 10,0mg/ml entsprechend 11,38 mg Gemcitabinhydrochlorid pro ml Wasser) und der pH-Wert mit q.s. 1 N Natronlauge / q.s. 1 N Salzsäure eingestellt.

Aus dem so gewonnenen Ansatz wurden mit Wasser für Injektionszwecke verdünnte Lösungen hergestellt und potentiometrisch deren pH-Wert bestimmt. Die hergestellten Lösungen wurden jeweils als 5ml-Aliquote in mit Durchstechstopfen dicht verschlossenen Vials abgefüllt. Diese Proben wurden nach Lagerung bei 2-8°C bzw. bei 25°C auf sichtbare Auskristallisation des Wirkstoffs geprüft ("+" Niederschlag; "-" klar und partikelfrei) und ihr pH-Wert bestimmt.

**Tabelle 1: Niederschlagbildung in 25°C und 2-8°C-gelagerter Gemcitabinlösungen (Anfangs-pH ca. 4,0)**

| | Gemcitabin Base (mg/ml) | pH final | nach 3 d (25°C) | nach 1 d (2-8°C) | nach 2 d (2-8°C) | nach 3 d (2-8°C) | nach 7 d (2-8°C) | nach 14 d (2-8°C) |
|---|---|---|---|---|---|---|---|---|
| 1. | 55,2 | 1,78 | - | + | + | + | + | + |
| 2. | 55,2 | 3,98 | - | ++++ | ++++ | ++++ | ++++ | ++++ |
| 3. | 46,0 | 4,14 | - | ++++ | ++++ | ++++ | ++++ | ++++ |
| 4. | 41,4 | 4,18 | - | ++++ | ++++ | ++++ | ++++ | ++++ |
| 5. | 36,8 | 4,20 | - | ++++ | ++++ | ++++ | ++++ | ++++ |
| 6. | 32,2 | 4,23 | - | +++ | +++ | +++ | +++ | +++ |
| 7. | 27,6 | 4,25 | - | ++ | ++ | ++ | ++ | ++ |
| 8. | 23,0 | 4,27 | - | + | + | + | + | + |
| 9. | 18,4 | 4,29 | - | - | + | + | + | + |
| 10. | 13,8 | 4,32 | - | - | + | + | + | + |
| 11. | 10,0 | 3,97 | - | - | - | - | - | - |
| 12. | 9,0 | 3,99 | - | - | - | - | - | - |
| 13. | 8,0 | 4,00 | - | - | - | - | - | - |
| 14. | 7,0 | 4,01 | - | - | - | - | - | - |
| 15. | 6,0 | 4,02 | - | - | - | - | - | - |
| 16. | 5,0 | 4,03 | - | - | - | - | - | - |
| 17. | 4,0 | 4,04 | - | - | - | - | - | - |
| 18. | 3,0 | 4,06 | - | - | - | - | - | - |
| 19. | 2,0 | 4,08 | - | - | - | - | - | - |
| 20. | 1,0 | 4,10 | - | - | - | - | - | - |

Die in Tabelle 1 zusammengefassten Daten zeigten überraschend, dass ohne zusätzliche Verwendung von löslichkeitssteigernden Hilfsstoffen klare partikelfreie Gemcitabinlösungen mit einem Gehalt von ca. 10 mg/ml Gemcitabin (ca. 11,4 mg Gemcitabinhydrochlorid pro ml Wasser) bei einem pH-Wert von ca. 4 bei 2-8°C Lagerung erhalten werden können.

### Beispiel 2

Der Gebrauchs- und Fachinformation des Gemzar®-Lyophilisats ist zu entnehmen, dass bei dessen Rekonstitution mit NaCl-Lösung pH-Werte im Bereich von 2,7 bis 3,3 erhalten werden. Die so erhaltenen Lösungen, die unmittelbar vor der Verabreichung an den Patienten aus dem Feststoff hergestellt werden müssen, können nur bei 25 °C, nicht aber bei Temperaturen von 2-8°C (kurzfristig) aufbewahrt werden. Aus dem Stand der Technik war zudem allgemein bekannt, dass die Löslichkeit von Gemcitabin in Wasser mit steigendem pH-Wert abnimmt.

Zur Bestimmung der tatsächlichen, pH-abhängigen Löslichkeit von Gemcitabin in wässrigen Lösungen wurden entsprechend Beispiel 1 konzentrierte Gemcitabin-Lösungen (c=52,7 mg Gemcitabin/ml entsprechend 60mg Gemcitabinhydrochlorid pro ml Wasser; 5 ml/Vial) hergestellt, deren pH-Wert mit q.s. 1 N Natronlauge auf pH-Werte von 2,0 bis 5,0 eingestellt wurde. Die so erhaltenen Lösungen wurden bei einer Lagertemperatur von 2-8°C gelagert.

Bei diesen Versuchen zeigte sich, dass Gemcitabinlösungen mit einem pH-Wert außerhalb eines Bereiches von ca. pH 3,5 eine sichtbare unerwünschte z.T. spontane (pH 5,0; 25°C) Auskristallisation zeigten ("+" Niederschlag; "-" klar und partikelfrei).

**Tabelle 2: Stabilität von konzentrierten Gemcitabin-Lösungen vs. Auskristallisation des Wirkstoffs**

| **Sichtbare Auskristallisation** | pH 2,0 | pH 2,5 | pH 3,0 | pH 3,5 | pH 4,0 | pH 4,5 | pH 5,0 |
|---|---|---|---|---|---|---|---|
| c=52,7mg Gemcitabin/ml | | | | | | | |
| nach 1 Tag (2-8°C) | + | - | - | - | +++ | ++++ | ++++ |
| nach 2 Tagen (2-8°C) | + | + | + | - | +++ | ++++ | ++++ |
| nach 3 Tagen (2-8°C) | + | + | + | - | +++ | ++++ | ++++ |
| nach 7 Tagen (2-8°C) | + | + | + | - | +++ | ++++ | ++++ |
| nach 14 Tagen (2-8°C) | + | + | + | - | +++ | ++++ | ++++ |

Aus den in Tabelle 2 zusammengestellten Daten ließ sich zweifelsfrei feststellen, dass ohne zusätzliche Verwendung von Löslichkeitssteigernden Hilfsstoffen bei 2-8°C gelagerte konzentrierte Gemcitabinsalzlösungen nur in einem sehr engen, sauren pH-Intervall sichtbar partikelfrei klar gelöst bleiben.

Zur weiteren Bestimmung der Temperatur-Abhängigkeit der Stabilität von erfindungsgemäßen Gemcitabin-Lösungen mit einem pH-Wert von ca. 4,0, wurden wie zuvor beschrieben Lösungen, deren pH-Wert mit 1 N Natronlauge eingestellt wurde, mit einer Gemcitabin-Konzentration von 10 mg/ml (in Wasser für Injektionszwecke) hergestellt. Diese wurden über einen Zeitraum von 12 Monaten gelagert. In dieser Zeit wurde die Wirkstoffkonzentration in regelmäßigen Abständen mittels HPLC bestimmt.

**Tabelle 3: Temperatur-abhängige Stabilität von Gemcitabin-Lösungen (c(Gemcitabin) = 10 mg/ml entsprechend 11,39 mg Gemcitabinhydrochlorid pro ml Wasser, pH 4,0)**

| Prüftakt | 5°C | 15°C | 25°C |
|---|---|---|---|
| | Gehalt | Gehalt | Gehalt |
| | (rel.%) | (rel.%) | (rel.%) |
| 0 mon | 100,0 | 100,0 | 100,0 |
| 3 mon | 99,7 | 99,6 | 97,1 |
| 6 mon | 99,4 | 99,2 | 94,1 |
| 9 mon | 99,2 | 98,8 | - - |
| 12 mon | 99,0 | 98,3 | - - |

| | | | |
|---|---|---|---|
| Sum. Imp. ≡ Anteil aller Verunreinigungen, bestimmt mittels HPLC | | | |

Bei Temperaturen bis zu 15°C zeigten die hier untersuchten Lösungen eine überraschende, pharmazeutisch akzeptable Lagerstabilität.

### Beispiel 3

Um die weitere Abhängigkeit der Stabilität von gepufferten Gemcitabin-Lösungen vom pH-Wert zu ermitteln, wurden Gemcitabin-Lösungen (c = 38 mg/ml) mit ausgewählten pH-Werten in einem Bereich von 3 bis 10 Stabilitätsuntersuchungen unterworfen. Dafür wurden jeweils Lösungen mit 10 mg/ml Gemcitabin (bezogen auf die Base), 10 mg/ml Mannitol, 5,0 mg/ml Natriumchlorid und Wasser für Injektionszwecke q. s. ad 1 ml Lösung hergestellt. Die Einstellung des pH-Wertes erfolgte unter Zugabe jeweils erforderlicher Mengen an 1 N Natronlauge oder 1 N Salzsäure. Zusätzlich wurden je nach pH-Wert die in folgender Tabelle 4 aufgeführten Puffer zur Stabilisierung des jeweiligen pH-Wertes eingesetzt. Die Bestimmung des pH-Wertes erfolgte potentiometrisch.

**Tabelle 4: Zur Stabilisierung des pH-Wertes der Gemcitabin-Lösungen verwendete Puffer**

| Testlösung | pH-Wert | Puffer |
|---|---|---|
| 1 | 3 | 0,625 mg/ml Natriumacetat |
| 2 | 4 | 0,625 mg/ml Natriumacetat |
| 3 | 5 | 0,625 mg/ml Natriumacetat |
| | | 0,625 mg/ml Natriumacetat + |
| 4 | 6 | 10 µmol/ml Dinatriumhydrogenphosphat-Dihydrat |
| | | 0,625 mg/ml Natriumacetat + |
| 5 | 7 | 10 gmol/ml Dinatriumhydrogenp4osphat-Dihydrat |
| | | 0,625 mg/ml Natriumacetat + |
| 6 | 8 | 10 µmol/ml Dinatriumhydrogenphosphat-Dihydrat |
| | | 0,625 mg/ml Natriumacetat + |
| 7 | 9 | 10 µmol/ml N-Methylglucamin |
| | | 0,625 mg/ml Natriumacetat + |
| 8 | 10 | 10 µmol/ml N-Methylglucamin |

Zur Herstellung der oben genannten Lösungen wurden 80 % des benötigten Wasser für Injektionszwecke vorgelegt. Die für den jeweiligen Ansatz benötigten, zuvor erwähnten Inhaltsstoffe mit Ausnahme des Wirkstoffes wurden nacheinander zugegeben und gelöst. Dann wurde Gemcitabinhydrochlorid zugefügt und klar gelöst. Der erwünschte pH-Wert wurde mit den erforderlichen Mengen an 1 N Natronlauge bzw. 1 N Salzsäure (Abweichung maximal +/- 0,2 pH-Stufe) eingestellt. Die Bestimmung des pH-Wertes erfolgte potentiometrisch. Der Ansatz wurde mit Wasser für Injektionszwecke aufgefüllt und der pH-Wert, wenn erforderlich, nochmals mit den erforderlichen Mengen an 1 N Natronlauge bzw. 1 N Salzsäure eingestellt (Abweichung maximal +/- 0,2 pH-Stufe).

Diese jeweils 50 ml Testlösungen wurden sterilfiltriert, jeweils 2 ml aseptisch in eine 2 ml Durchstechflasche abgefüllt, die Flaschen mit Durchstechstopfen verschlossen, luftdicht verbördelt und anschließend lichtgeschützt über die Dauer von einem Monat bei 55 °C "unter beschleunigten Bedingungen" gelagert.

Der Stabilitätsparameter "pH-Wert" wurde potentiometrisch und die Stabilitätsparameter "Gehalt und Reinheit" per HPLC bestimmt.

Die Ergebnisse der "unter beschleunigten Bedingungen" bei 55 °C erfolgten Untersuchungen sind in der nachfolgenden Tabelle 5 zusammengefasst. Die "Stabilität der Lösung" ist immer als Stabilität des Gemcitabins in Lösung zu verstehen, d.h. als langfristige Konstanz der Konzentration der Ausgangsverbindung nach Inlösungbringen.

**Tabelle 5: Stabilitäten von Gemcitabin-Lösungen bei pH = 3-10 bei 55 °C**

| Testlösung | pH-Wert | Start Gehalt Gemcitabin [rel.%] | 1 mon Gehalt Gemcitabin [rel.%] | 1 mon Gehaltsverlust ^{*1} [rel.%] | Start Sum. Imp.^{*2} [%] | 1 mon Sum. Imp.^{*2} [%] |
|---|---|---|---|---|---|---|
| 1 | 3 | 100,0 | 60,2 | -39,8 | 0,030 | 18,770 |
| 2 | 4 | 100,0 | 84,5 | -15,5 | 0,025 | 8,353 |
| 3 | 5 | 100,0 | 94,0 | -6,0 | 0,025 | 1,459 |
| 4 | 6 | 100,0 | 99,4 | -0,6 | 0,026 | 0,559 |
| 5 | 7 | 100,0 | 99,1 | -0,9 | 0,027 | 0,478 |
| 6 | 8 | 100,0 | 99,3 | -0,7 | 0,027 | 0,475 |
| 7 | 9 | 100,0 | 97,0 | -3,0 | 0,027 | 0,476 |
| 8 | 10 | 100,0 | 97,0 | -3,0 | 0,035 | 1,138 |

| | | | | | | |
|---|---|---|---|---|---|---|
| ^{*1} Gehaltsverlust Gemcitabin relativ zum Ausgangswert. ^{*2} Sum. Imp. ≡ Summe der Verunreinigungen. | | | | | | |

Der pH-Wert der Testlösungen mit einem pH-Wert von 5-10 blieb mit einer Toleranz von maximal +/- 0,2 pH-Stufe über den Zeitraum der beschleunigten Stabilitätsuntersuchung nahezu unverändert. Die Testlösungen mit einem pH-Wert von 3 und 4 zeigten einen pH-Anstieg von bis zu einer pH-Stufe, der mit dem Zersetzungsgrad des Wirkstoffs in den Testlösungen korreliert.

Die in Tabelle 5 zusammengefassten Daten der Versuchsreihe zeigen eindrucksvoll, dass das Stabilitätsoptimum von Gemcitabin in wässriger Lösung (c = 10 mg/ml) nicht im stark sauren, sondern im pH-Bereich von 4 bis 10 liegt, vorzugsweise bei pH 6 bis 9, insbesondere bei pH 7 bis 9. Ein besonders günstiger pH-Bereich liegt um pH 8.

Verglichen mit der Stabilität herkömmlicher Infusionslösungen mit deren pH-Werten um 3 (2,7 bis 3,3) kann überraschenderweise in einem pH-Bereich von ca. 4,0 bis ca. 10,0 der Gehaltsverlust des Gemcitabins um das ca. 3 - bis 57-fache und eine Zunahme der Summe an Verunreinigungen um das ca. 13- bis 40-fache verringert werden.

### Beispiel 4

Eine gepufferte Gemcitabin-Lösung mit einem pH-Wert von ca. 8,0 (7,8-8,2) mit der nachfolgend aufgeführten Zusammensetzung (Testlösung 9) wurde einer Stabilitätsuntersuchung unterzogen.

### Zusammensetzung der Testlösung 9:

| | |
|---|---|
| Gemcitabin | 10,0 mg/ml (bezogen auf die Base) |
| Mannitol | 10,0 mg/ml |
| Natriumacetat | 0,625 mg/ml |
| Natriumchlorid | 5,0 mg/ml |
| 1 N Natronlauge evtl. 1 N Salzsäure q. s. ad pH 8,0 | |
| Wasser für Injektionen q. s. ad 1 ml Lösung | |

Zur Herstellung der oben genannten Lösung (Testlösung 9) wurden etwa 80 % des benötigten Wasser für Injektionszwecke (20-25 °C) im Ansatzbehälter vorgelegt. Mannitol, Natriumacetat und Natriumchlorid wurden nacheinander zugegeben und klar gelöst. Dann wurde Gemcitabinhydrochlorid zugefügt und klar gelöst. Der erwünschte pH-Wert wurde mit den erforderlichen Mengen an 1 N Natronlauge bzw. 1 N Salzsäure eingestellt (Abweichung maximal +/- 0,2 pH-Stufe). Die Bestimmung des pH-Wertes erfolgte potentiometrisch. Der Ansatz wurde mit Wasser für Injektionszwecke aufgefüllt und der pH-Wert, wenn erforderlich, nochmals mit den erforderlichen Mengen an 1 N Natronlauge bzw. 1 N Salzsäure eingestellt (Abweichung +/- 0,2 pH-Stufe).

500 ml dieser Testlösung wurden sterilfiltriert, jeweils 2 ml aseptisch in eine 2 ml Durchstechflasche abgefüllt, die Flaschen mit Durchstechstopfen verschlossen, luftdicht verbördelt und anschließend lichtgeschützt bei 25 °C, 40 °C oder 60 °C über die Dauer von 18 Monaten gelagert.

Der Stabilitätsparameter "pH-Wert" wurde potentiometrisch und die Stabilitätsparameter "Gehalt und Reinheit" per HPLC bestimmt.

Die neben 25 °C auch "unter beschleunigten Bedingungen" bei 40 °C und 60 °C erfolgte Untersuchung zur Ermittlung der Lagerstabilität der Gemcitabin-Lösungen ergab folgende in Tabelle 6 zusammengefasste Ergebnisse.

**Tabelle 6: Stabilität von Gemcitabin-Lösungen mit pH = 8,0 bei 25°C, 40°C und 60°C**

| | 25°C | 25°C | 40°C | 40°C | 60°C | 60°C |
|---|---|---|---|---|---|---|
| | Gehalt | Sum. Imp.^{*} | Gehalt | Sum. Imp.^{*} | Gehalt | Sum. Imp.^{*} |
| | [rel.%] | [%] | [rel.%) | [%] | [rel.%] | [%] |
| Start | 100,0 | 0,040 | 100,0 | 0,040 | 100,0 | 0,040 |
| 1 mon | 100,01 | 0,058 | 100,02 | 0,104 | 99,62 | 0,381 |
| 2 mon | 100,04 | 0,069 | 100,02 | 0,174 | 98,41 | 0,938 |
| 3 mon | 99,79 | 0,076 | 100,04 | 0,216 | 97,53 | 1,217 |
| 6 mon | 99,61 | 0,095 | 97,22 | 0,439 | 94,79 | 2,745 |
| 9 mon | 99,23 | 0,184 | 98,83 | 0,668 | -- | -- |
| 12 mon | 99,55 | 0,196 | 98,31 | 0,951 | -- | -- |
| 18 mon | 99,02 | 0,279 | -- | -- | -- | -- |

| | | | | | | |
|---|---|---|---|---|---|---|
| ^{*} "Sum. Imp." bedeutet Summe der Verunreinigungen. | | | | | | |

Über die Dauer von 18 Monaten bei einer Lagertemperatur von 25 °C blieben die Lösungen klar, farblos und frei von sichtbaren Partikeln und Kristallbildung.

Der pH-Wert blieb mit einer Toleranz von maximal +/- 0,2 pH-Stufe (25 °C), bzw. +/- 0,3 pH-Stufe (55 °C) über den Zeitraum der Stabilitätsuntersuchung nahezu unverändert.

Eine regressive Extrapolation der in Tabelle 6 angegebenen Daten ergab folgende in Tabelle 7 aufgeführte Lagerstabilitäten.

**Tabelle 7 : Stabilität von Gemcitabin-Lösungen mit pH = 8,0 bei 25°C, 40°C und 60°C bei einer Lagerzeit von 1-4 Jahren**

| | 25°C | 25°C | 40°C | 40°C | 60°C | 60°C |
|---|---|---|---|---|---|---|
| | Gehalt | Sum. Imp.^{*} | Gehalt | Sum. Imp.^{*} | Gehalt | Sum. Imp.^{*} |
| | [rel.%] | [%] | [rel.%] | [%] | [rel.%] | [%] |
| 12 mon | 99,34 | 0,200 | 97,92 | 0,916 | 89,43 | 5,415 |
| 24 mon | 98,67 | 0,361 | 95,80 | 1,818 | 78,64 | 10,85 |
| 36 mon | 97,99 | 0,521 | 93,68 | 2,719 | 67,85 | 16,29 |
| 48 mon | 97,32 | 0,682 | 91,56 | 3,621 | 57,05 | 21,73 |

| | | | | | | |
|---|---|---|---|---|---|---|
| ^{*} "Sum. Imp." bedeutet Summe der Verunreinigungen. | | | | | | |

Die Daten zeigen, dass Gemcitabin in einer Konzentration von 10 mg/ml in wässriger Lösung bei einem pH-Wert von ca. 8 eine unerwartet hohe Lagerstabilität bei einer Lagertemperatur von 25 °C aufweist. Ferner sind diese Lösungen bei einer Lagertemperatur von 25°C über eine Dauer von mindestens 3 Jahren stabil.

### Beispiel 5

Zur Ermittlung der Konzentrationsabhängigkeit der Stabilität von Gemcitabin-Lösungen wurden nachfolgend angegebene Lösungen mit einem pH-Wert von ca. 8,0 (7,8-8,2) hergestellt und analysiert.

### Testlösungen 10-12:

Es wurden jeweils Lösungen mit
- 0,05 mg/ml (= 50 µg/ml) Gemcitabin-Base
- 0,25 mg/ml (= 250 µg/ml) Gemcitabin-Base
- 1,00 mg/ml Gemcitabin-Base
mit q. s. Wasser für Injektionszwecke ad 1 ml hergestellt. Der pH-Wert wurde mit q. s. Salzsäure bzw. Natronlauge auf ca. 8,0 (7,8-8,2) eingestellt und mit 2,0 mg/ml Trometamol stabilisiert.

Die erforderlichen Gemcitabin-Mengen wurden in 80 % des erforderlichen Wasser für Injektionszwecke aufgelöst und dann das Trometamol darin gelöst. Der pH-Wert wurde mit q. s. 1 N Salzsäure bzw. 1 N Natronlauge auf ca. 8,0 (+/- 0,2) eingestellt. Die Ansätze wurden mit Wasser für Injektionszwecke aufgefüllt und der pH-Wert, wenn erforderlich, wie zuvor beschrieben auf 8,0 (+/- 0,2) eingestellt. Die Ansätze wurden sterilfiltriert, aseptisch zu jeweils 5 ml pro Vial abgefüllt, und die Vials mit Injektionsstopfen und Bördelkappen luftdicht verschlossen.

Die Chargen wurden lichtgeschützt bei 40 °C (unter "beschleunigten Bedingungen") gelagert. Die Gehaltsbestimmung erfolgte mittels HPLC.

Die Bestimmung des pH-Wertes erfolgte potentiometrisch. Die Abweichungen innerhalb der Testdauer lagen bei +/- 0,2 pH-Stufe.

Die Ergebnisse der Untersuchungen sind in nachfolgender Tabelle 8 zusammengefasst.

**Tabelle 8 : Konzentrationsabhängigkeit der Stabilität von Gemcitabin-Lösungen**

| Gemcitabinkonzentration | Gemcitabinkonzentration | Gemcitäbinkonzentration |
|---|---|---|
| 0,05 mg/ml | 0,25 mg/ml | 1,00 mg/ml . |
| Start: 100% | Start: 100% | Start: 100% |
| 1 mon: 99,8% | 1 mon: 100,0% | 1 mon: 100,0% |
| 3 mon: 99,3% | 3 mon: 99,5% | 3 mon: 99,7% |
| 6 mon: 98,3% | 6 mon: 98,8% | 6 mon : 99,3% |

Wie deutlich aus Tabelle 8 zu erkennen ist, sind die erfindungsgemäßen Gemcitabin-Lösungen selbst bei Konzentrationen bis hinunter zu c = 0,05 mg/ml lagerstabil.

### Beispiele 6-18

Zur Bestimmung der Stabilität von erfindungsgemäßen pH-eingestellten Gemcitabin-Lösungen, zum Teil umfassend weitere Hilfsstoffe, Konservierungs- und Antioxidationsmittel, wurden entsprechende Lösungen, wie schon zuvor beschrieben, hergestellt, über 12 Monate gelagert, und der Wirkstoffgehalt per HPLC bestimmt. Aus diesen Daten konnte die zu erwartende Lagerdauer (t₉₅), bei der noch ein Wirkstoffgehalt von 95% der Anfangskonzentration vorliegt, inter- bzw. extrapoliert werden. Die Ergebnisse dieser Untersuchungen sind in Tabelle 9 zusammengefasst.

**Tabelle 9: t₉₅ - Stabilitäten hergestellter Gemcitabinlösungen**

| Bsp.: | 6 | 7 | 8 | 9 | 10 | 11 | 12 | 13 | 14 | 15 | 16 | 17 | 18 |
|---|---|---|---|---|---|---|---|---|---|---|---|---|---|
| Einwaagen [mg/ml] | | | | | | | | | | | | | |
| Gemcitabin | 10,0 | 10,0 | 10,0 | 10,0 | 10,0 | 10,0 | 10,0 | 10,0 | 10,0 | 10,0 | 10,0 | 10,0 | 10,0 |
| Natronlauge 1N / Salzsäure 1N | q.s. | q.s. | q.s. | q.s. | q.s. | q.s. | q.s. | q.s. | q.s. | q.s. | q.s. | q.s. | q.s. |
| Natriumchlorid | 6,50 | - | - | 6,50 | 5,50 | 3,50 | 6,50 | 3,50 | 4,00 | 5,00 | - | 5,0 | - |
| Mannitol | - | 40,0 | - | | - | - | - | - | - | 10,0 | - | - | - |
| Glucose Monohydrat | - | - | 45,0 | - | - | - | - | - | - | - | - | - | - |
| Trometamol | - | - | - | 0,25 | - | - | - | - | - | - | - | - | - |
| Natriummetabisulfit | - | - | - | - | - | 2,00 | | 2,00 | - | - | - | - | - |
| Natriumedetat | - | - | - | - | - | - | 0,10 | 0,10 | - | - | - | - | - |
| Trometamol | - | - | - | - | - | 2,00 | - | 2,00 | 10,0 | - | - | - | - |
| Natriumazetat | - | - | - | - | - | - | - | - | - | 0,625 | - | - | 3,25 |
| Methylparaben | - | - | - | - | 1,30 | - | - | - | - | - | - | - | - |
| Propylparaben | - | - | - | - | 0,20 | - | - | - | - | - | - | - | - |
| Benzylalkohol | - | - | - | - | - | - | - | - | 10,0 | - | - | - | - |
| Wasser für Injektionszwecke | q.s. | q.s. | q.s. | q.s. | q.s. | q.s. | q.s. | q.s. | q.s. | q.s. | q.s. | q.s. | q.s. |
| pH-Wert | 8.1 | 8.2 | 8.0 | 8.0 | 7.8 | 7.9 | 8.0 | 8.1 | 7.9 | 8,3 | 3,9 | 4,1 | 3,5 |
| t₉₅* (mon; 2-8°C) | - | - | - | - | - | - | - | - | - | - | 56 | 52 | 17 |
| t₉₅* (mon; 15°C) | - | - | - | - | - | - | - | - | - | - | 36 | 28 | 8 |
| t₉₅* (mon; 25°C) | 108 | 100 | 85 | 77 | 110 | 82 | 57 | 87 | 91 | 146 | 5 | 4 | 2 |
| t₉₅* (mon; 40°C) | 20 | 20 | 18 | 17 | 19 | 18 | 16 | 17 | 19 | 22 | - | - | 0,5 |
| t₉₅* (mon; 60°C) | 6 | 7 | 6 | 5 | 6 | 5 | 4 | 6 | 5 | 6 | - | - | - |

| | | | | | | | | | | | | | |
|---|---|---|---|---|---|---|---|---|---|---|---|---|---|
| * gerundet | | | | | | | | | | | | | |

Die hier ermittelten Stabilitätsdaten belegen eindrucksvoll, dass bei 2-8°C gelagerte (pH ca. 4) als auch bei 25°C gelagerte, erfindungsgemäße Gemcitabin-Lösungene-(pH ca. 8) über eine Dauer von mindestens 3 Jahren stabil sind.

### Beispiele 19-63

Es wurden wässrige Lösungen folgender Zusammensetzung hergestellt:

| Beispiel | Gehalt Gemcitabin [mg/ml] | Alkalisierungs-/Ansäuerungsmittel | pH-Wert | Puffer [m/V] | Hilfsstoff [m/V] |
|---|---|---|---|---|---|
| 19 | 0,06 | TRIS | 8,2 | q. s. TRIS | - |
| 20 | 1,0 | Natronlauge | 8,3 | | 0,9% NaCl |
| 21 | 5,0 | Natronlauge | 8,7 | | 0,9% NaCl |
| 22 | 5,0 | Natronlauge | 8,7 | 0,05 % Meglumin | 0,9% NaCl |
| 23 | 10,0 | TRIS | 5,0 | 0,02 % Natriumdihydrogenphosphat | 5 % Mannitol |
| 24 | 10,0 | Meglumin | 8,0 | | 5 % Glucose |
| 25 | 16,0 | Natronlauge/ | 8,0 | | |
| | | Salzsäure | | | |
| 26 | 10,0 | Natronlauge/ | 8,0 | | 0,65% |
| | | Salzsäure | | | Natriumchlorid |
| 27 | 10,0 | Natronlauge/ | 8,0 | | 4,0% Mannitol |
| | | Salzsäure | | | |
| 28 | 10,0 | Natronlauge/ | 8,0 | | 4,5% Glucose |
| | | Salzsäure | | | |
| 29 | 10,0 | Natronlauge/ | 8,0 | 0,025% Trometamol | 0,65% |
| | | Salzsäure | | | Natriumchlorid |
| 30 | 10,0 | Natronlauge/ | 8,0 | | 0,55% |
| | | Salzsäure | | | Natriumchlorid, |
| | | | | | 0,13% |
| | | | | | Methylparaben, |
| | | | | | 0,02% |
| | | | | | Propylparaben |
| 31 | 10,0 | Natronlauge/ | 8,0 | 0,5% Trometamol | 0,55% |
| | | Salzsäure | | | Natriumchlorid, |
| | | | | | 0,20% |
| | | | | | Natriummetabisulfit |
| 32 | 10,0 | Natronlauge/ | 8,0 | | 0,65% |
| | | Salzsäure | | | Natriumchlorid |
| | | | | | 0,01% |
| | | | | | Natriumedetat |
| 33 | 10,0 | Natronlauge/ | 8,0 | 0,5% Trometamol | 0,25% |
| | | Salzsäure | | | Natriumchlorid, |
| | | | | | 0,20% |
| | | | | | Natriummetabisulfit, |
| | | | | | 0,01% |
| | | | | | Natriumedetat |
| 34 | 10,0 | Natronlauge/ | 8,0 | | 0,40% |
| | | Salzsäure | | | Natriumchlorid, |
| | | | | | 1,0% |
| | | | | | Benzylalkohol |
| 35 | 10,0 | Meglumin | 9,0 | | 0,70% |
| | | | | | Natriumchlorid |
| 36 | 10,0 | Meglumin | 10,0 | | 0,70% |
| | | | | | Natriumchlorid |
| 37 | 10,0 | Dinatriumhydrogenphosphat Dihydrat | 8,0 | | 0,65% |
| | | | | | Natriumchlorid |
| 38 | 16,0 | Natronlauge | 10,0 | | |
| 39 | 16,0 | Natronlauge | 5,0 | | |
| 40 | 16,0 | Natronlauge | 5,0 | 0,0625% | |
| | | | | Natriumazetat | |
| 41 | 0,05 | Natronlauge/ | 10,0 | 0,2% Meglumin | |
| | | Salzsäure | | | |
| 42 | 0,05 | Meglumin | 10,0 | | |
| 43 | 0,05 | Meglumin | 9,0 | | 0,90% |
| | | | | | Natriumchlorid |
| 44 | 0,05 | Trometamol | 8,0 | | |
| 45 | 16,0 | Natronlauge/ | 10,0 | | |
| | | Salzsäure | | | |
| 46 | 10,0 | Natronlauge/ | 4,0 | | |
| | | Salzsäure | | | |
| 47 | 10,0 | Natronlauge/ | 4,0 | | |
| | | Milchsäure | | | |
| 48 | 10,0 | Natronlauge/ | 4,0 | | |
| | | Milchsäure | | | |
| 49 | 10,0 | Natronlauge/ | 5,0 | | |
| | | Essigsäure | | | |
| 50 | 10,0 | Natronlauge/ | 4,0 | 0,05%Natriumcitrat | |
| | | Salzsäure | | | |
| 51 | 10,0 | Natronlauge/ | 4,0 | 0,05%Natriumlactat | |
| | | Milchsäure | | | |
| 52 | 5,0 | Natronlauge/ | 4,0 | 0,04%Natriumlactat | 0,55% |
| | | Milchsäure | | | Natriumchlorid |
| 53 | 5,0 | Natronlauge/ | 5,0 | 0,45%Natriumazetat | 0,55% |
| | | Essigsäure | | | Natriumchlorid |
| 5.4 | 5,0 | Natronlauge/ | 4,0 | | 0,55% |
| | | Salzsäure | | | Natriumchlorid |
| 55 | 5,0 | Natronlauge/ | 4,0 | | 0,60% |
| | | Milchsäure | | | Natriumchlorid |
| 56 | 5,0 | Natronlauge/ | 4,0 | | 0,55% |
| | | Milchsäure | | | Natriumchlorid |
| 57 | 5,0 | Natronlauge/ | 4,0 | | 0,60% |
| | | Essigsäure | | | Natriumchlorid |
| 58 | 0,5 | Natronlauge/ | 4,0 | | |
| | | Essigsäure | | | |
| 59 | 0,05 | Natronlauge/ | 4,0 | | 0,90% |
| | | Essigsäure | | | Natriumchlorid |
| 60 | 0,05 | Natronlauge/ | 4,0 | | |
| | | Essigsäure | | | |
| 61 | 10,0 | Natronlauge/ | 3,5 | | |
| | | Essigsäure | | | |
| 62 | 1,0 | Natronlauge/ | 3,5 | | |
| | | Essigsäure | | | |
| 63 | 0,05 | Natronlauge/ | 3,5 | | |
| | | Essigsäure | | | |

## Patentansprüche

1. Pharmazeutische Zusammensetzungen umfassend ein Säureadditionssalz der Gemcitabin-Base mit einer anorganischen Säure in der Form einer gebrauchsfertigen Lösung, die nicht unmittelbar vor der Verabreichung an den Patienten aus einem Feststoff rekonstituiert wird, wobei der pH-Wert der Lösung oberhalb von 3,5 liegt und die Gemcitabin-Konzentration wenigstens 0,05 mg/ml beträgt.

2. Pharmazeutische Zusammensetzungen umfassend Gemcitabinhydrochlorid in der Form einer gebrauchsfertigen Lösung, die nicht unmittelbar vor der Verabreichung an den Patienten aus einem Feststoff rekonstituiert wird, wobei der pH-Wert der Lösung oberhalb von 3,5 liegt und die Gemcitabin-Konzentration wenigstens 0,05 mg/ml beträgt.

3. Pharmazeutische Zusammensetzung nach Anspruch 1 oder 2, wobei der pH-Wert der Lösung in einem Bereich von 3,5 bis 10,0 liegt.

4. Pharmazeutische Zusammensetzung nach einem der Ansprüche 1-3, wobei die Gemcitabin-Konzentration zwischen 0,05 mg/ml und 16,0 mg/ml liegt.

5. Pharmazeutische Zusammensetzung nach einem der Ansprüche 1-4, wobei die Gemcitabin-Konzentration 10 mg/ml beträgt.

6. Pharmazeutische Zusammensetzung nach einem der Ansprüche 1-5, wobei der pH-Wert der Lösung mit dem physiologischen Gewebe- und Blut-pH-Wert von 7,35 bis 7,55 übereinstimmt.

7. Pharmazeutische Zusammensetzung nach einem der Ansprüche 1-6, wobei die Einstellung des pH-Wertes der Lösung durch Mischen der Gemcitabin-Base mit einem physiologisch annehmbaren Säureadditionssalz davon, vorzugsweise mit Gemcitabinhydrochlorid, erfolgt.

8. Pharmazeutische Zusammensetzung nach einem der Ansprüche 1-7, wobei die Einstellung des pH-Wertes der Lösung mit mindestens einem physiologisch annehmbaren Ansäuerungsmittel erfolgt.

9. Pharmazeutische Zusammensetzung nach Anspruch 8, wobei das Ansäuerungsmittel Salzsäure ist.

10. Pharmazeutische Zusammensetzung nach einem der Ansprüche 1-6, wobei die Einstellung des pH-Wertes der Lösung mit mindestens einem physiologisch annehmbaren Alkalisierungsmittel erfolgt.

11. Pharmazeutische Zusammensetzung nach Anspruch 10, wobei das Alkalisierungsmittel Natriumhydroxid ist.

12. Pharmazeutische Zusammensetzung nach einem der Ansprüche 1-6, wobei die Einstellung des pH-Wertes der Lösung mit einem Puffer erfolgt.

13. Pharmazeutische Zusammensetzung nach Anspruch 12, wobei als Puffer Tris(hydroxymethyl-)aminomethan, 1-Desoxy-(methylamino)-D-glucitol, Natriumacetat und/oder Dinatriumhydrogenphosphat verwendet wird.

14. Pharmazeutische Zusammensetzung nach einem der Ansprüche 1-13, die gegebenenfalls zusätzlich mindestens einen tonisierenden und/oder konservierenden Hilfsstoff umfasst.

15. Verwendung der pharmazeutischen Zusammensetzungen nach einem der Ansprüche 1-12 zur Herstellung eines Arzneimittels zur Behandlung einer Tumorerkrankung.

16. Verfahren zur Herstellung der pharmazeutischen Zusammensetzungen gemäß einem der Ansprüche 1-14, wobei als Wirkstoff ein Gemcitabin-Säureadditionssalz verwendet wird.

17. Verfahren gemäß Anspruch 16, wobei das Gemcitabin-Säureadditionssalz Gemcitabinhydrochlorid ist.

## Claims

1. Pharmaceutical compositions comprising an acid addition salt of the gemcitabine base with an inorganic acid, in the form of a ready-to-use solution that is not reconstituted from a solid immediately prior to administration to the patient, wherein the pH value of the solution is above 3.5 and the gemcitabine concentration is at least 0.05 mg/ml.

2. Pharmaceutical compositions comprising gemcitabine hydrochloride in the form of a ready-to-use solution that is not reconstituted from a solid immediately prior to administration to the patient, wherein the pH value of the solution is above 3.5 and the gemcitabine concentration is at least 0.05 mg/ml.

3. Pharmaceutical composition according to claim 1 or 2, wherein the pH value of the solution is in a range from 3.5 to 10.0.

4. Pharmaceutical composition according to any one of claims 1 to 3, wherein the gemcitabine concentration is from 0.05 mg/ml to 16.0 mg/ml.

5. Pharmaceutical composition according to any one of claims 1 to 4, wherein the gemcitabine concentration is 10 mg/ml.

6. Pharmaceutical composition according to any one of claims 1 to 5, wherein the pH value of the solution corresponds to the physiological pH value of tissue and blood of from 7.35 to 7.55.

7. Pharmaceutical composition according to any one of claims 1 to 6, wherein the pH value of the solution is adjusted by mixing the gemcitabine base with a physiologically acceptable acid addition salt thereof, preferably with gemcitabine hydrochloride.

8. Pharmaceutical composition according to any one of claims 1 to 7, wherein the pH value of the solution is adjusted with at least one physiologically acceptable acidifying agent.

9. Pharmaceutical composition according to claim 8, wherein the acidifying agent is hydrochloric acid.

10. Pharmaceutical composition according to any one of claims 1 to 6, wherein the pH value of the solution is adjusted with at least one physiologically acceptable alkalinising agent.

11. Pharmaceutical composition according to claim 10, wherein the alkalinising agent is sodium hydroxide.

12. Pharmaceutical composition according to any one of claims 1 to 6, wherein the pH value of the solution is adjusted with a buffer.

13. Pharmaceutical composition according to claim 12, wherein tris(hydroxymethyl)aminomethane, 1-deoxy-(methylamino)-D-glucitol, sodium acetate and/or disodium hydrogen phosphate is used as the buffer.

14. Pharmaceutical composition according to any one of claims 1 to 13 optionally additionally comprising at least one tonicity-adjusting and/or preserving auxiliary substance.

15. Use of the pharmaceutical compositions according to any one of claims 1 to 12 in the preparation of a medicament for treating a tumour disease.

16. Process for the preparation of the pharmaceutical compositions according to any one of claims 1 to 14, wherein a gemcitabine acid addition salt is used as the active ingredient.

17. Process according to claim 16, wherein the gemcitabine acid addition salt is gemcitabine hydrochloride.

## Revendications

1. Compositions pharmaceutiques comprenant un sel d'addition d'acide de la base gemcitabine avec un acide inorganique sous forme d'une solution prête à l'emploi, qui n'est pas reconstituée à partir d'une substance solide immédiatement avant l'administration aux patients, dans lesquelles la valeur du pH de la solution est supérieure à 3,5 et la concentration en gemcitabine est d'au moins 0,05 mg/ml.

2. Compositions pharmaceutiques comprenant du chlorhydrate de gemcitabine sous forme d'une solution prête à l'emploi, qui n'est pas reconstituée à partir d'une substance solide immédiatement avant l'administration aux patients, dans lesquelles la valeur du pH de la solution est supérieure à 3,5 et la concentration en gemcitabine est d'au moins 0,05 mg/ml.

3. Composition pharmaceutique selon la revendication 1 ou 2, dans laquelle la valeur du pH de la solution est dans une plage de 3,5 à 10,0.

4. Composition pharmaceutique selon l'une quelconque des revendications 1 à 3, dans laquelle la concentration en gemcitabine est comprise entre 0,05 mg/ml et 16,0 mg/ml.

5. Composition pharmaceutique selon l'une quelconque des revendications 1 à 4, dans laquelle la concentration en gemcitabine est de 10 mg/ml.

6. Composition pharmaceutique selon l'une quelconque des revendications 1 à 5, dans laquelle la valeur du pH de la solution coïncide avec la valeur du pH physiologique tissulaire et sanguine de 7,35 à 7,55.

7. Composition pharmaceutique selon l'une quelconque des revendications 1 à 6, dans laquelle l'ajustement de la valeur du pH de la solution se réalise en mélangeant la base gemcitabine avec un sel d'addition d'acide, acceptable sur le plan physiologique, de préférence avec le chlorhydrate de gemcitabine.

8. Composition pharmaceutique selon l'une quelconque des revendications 1 à 7, dans laquelle l'ajustement de la valeur du pH de la solution se réalise avec au moins un agent d'acidification acceptable sur le plan physiologique.

9. Composition pharmaceutique selon la revendication 8, dans laquelle l'agent d'acidification est l'acide chlorhydrique.

10. Composition pharmaceutique selon l'une quelconque des revendications 1 à 6, dans laquelle l'ajustement de la valeur du pH de la solution se réalise avec au moins un agent d'alcalinisation acceptable sur le plan physiologique.

11. Composition pharmaceutique selon la revendication 10, dans laquelle l'agent d'alcalinisation est l'hydroxyde de sodium.

12. Composition pharmaceutique selon l'une quelconque des revendications 1 à 6, dans laquelle l'ajustement de la valeur du pH de la solution se réalise avec un tampon.

13. Composition pharmaceutique selon la revendication 12, dans laquelle on emploie le tris(hydroxyméthyl)aminométhane, le 1-désoxy-(méthylamino)-D-glucitol, l'acétate de sodium et/ou l'hydrogénophosphate disodique en tant que tampon.

14. Composition pharmaceutique selon l'une quelconque des revendications 1 à 13, qui comprend éventuellement en sus au moins un adjuvant tonifiant et/conservateur.

15. Utilisation des compositions pharmaceutiques selon l'une quelconque des revendications 1 à 12 pour préparer un médicament destiné au traitement d'une maladie tumorale.

16. Procédé de préparation des compositions pharmaceutiques selon l'une quelconque des revendications 1 à 14, dans lequel un sel d'addition d'acide de gemcitabine est employé en tant que substance active.

17. Procédé selon la revendication 16, dans lequel le sel d'addition d'acide de gemcitabine est le chlorhydrate de gemcitabine.
